# EUROPEAN PATENT APPLICATION

(11) **EP 4 641 054 A1**
(43) Date of publication of application: **29.10.2025**
(21) Application number: 24172283.4
(22) Date of filing: 24.04.2024
(51) Int. Cl.: F16K 3/24, A61M 39/22, F16K 3/26, F16K 27/04

(54) **VALVE DEVICE**

(71) Applicant: Sartorius Stedim Biotech GmbH, 37079 Göttingen (DE)
(72) Inventor: PURSHOUSE, Martin, 37079 Göttingen (DE); JAKOB, Fabian, 37079 Göttingen (DE); FRIESE, Thomas, 37079 Göttingen (DE); LOEWE, Thomas, 37079 Göttingen (DE)
(74) Representative: Prinz & Partner mbB

(57) **Abstract**

A valve device (10), especially for use with a small volume filtration device or for use in a fluid line in a pharmaceutical or biotechnology application, comprises a housing component (12) including a fluid chamber (22) and a fluid transfer opening (14) for establishing fluid communication with a first external fluid component. The valve device (10) further comprises a valve body (32) received in the housing component (12). The valve body (32) includes a hollow section (34), a first end cooperating with the fluid transfer opening (14) inside the housing component (12), a second end for connecting the valve device (10) to a second external fluid component, at least one first valve body opening (38) connecting the hollow section (34) with the fluid chamber (22) of the housing component (12), a second valve body opening (42) for fluid transfer from or to the second external fluid component, and a first thread structure formed at an outer wall of the valve body (32). The valve body (32) is translationally movable along an axis in the housing component (12) between an open position and a closed position. In the closed position of the valve body (32) the second end closes the fluid transfer opening (14) of the housing component (12). In the open position of the valve body (32) the second end is removed from the fluid transfer opening (14) of the housing component (12) so that a fluid connection is established between the fluid transfer opening (14) and the fluid chamber (22). The valve device (10) further comprises an actuator ring element (56) for actuating the valve device (10). The actuator ring element (56) is rotatably mounted on the housing component (12) and includes an accessible structure that can be engaged manually or by a tool to rotate the actuator ring element (56), and a second thread structure formed at an inner wall of the actuator ring element (56). The second thread structure engages the first thread structure of the valve body (32), so that a rotation of the actuator ring element (56) relative to the housing component (12) and the valve body (32) causes the valve body (32) to translationally move in the housing component (12). The valve device (10) further comprises means for sealing the fluid chamber (22) from the outside of the valve device (10).

## Description

The invention relates to a valve device, especially for use with a small volume filtration device or for use in a fluid line in a pharmaceutical or biotechnology application.

Valves used in connection with filter elements or pipes typically have a valve body and a valve seat that are produced separately and are assembled afterwards. Such valves are mostly opened and closed by twisting the valve body or the valve seat, so that the valve body pushes itself out of or against the valve seat, respectively. This translational movement can be achieved by cams or pins being guided in slanted slots, for example (see e.g. WO 03/090843 A1, EP 1 321 699 B1). Most of these valves have one or more O-rings to seal against media and pressure inside of the filter devices or the pipes.

Further, valves are known with a third component, especially a separate ring element. With such a ring element, various features can be implemented, e.g. preventing disassembly of the valve device. Most of these known valves have connectors for a tube connection and must be twisted to be opened and closed (see e.g. US 11 666 743 B2, US 2008/087860 A1). By twisting these valves, the tubes are also twisted. However, a twist of connected components can bring torsion to the system and lead to leakage.

Other known valves with non-twisting connectors have complicated designs, typically with gaps or other apertures on the outside (e.g. due to open guides), resulting in high manufacturing costs and outer surfaces that cannot easily be wiped with a sterile cloth. The latter is problematic when a valve is to be used in a particle-free area such as a cleanroom.

An example of a known valve device is shown in EP 0 691 492 A1. The valve device can be inserted into a water pipe or attached to the end of it. It has a hollow piston that moves within a guiding conduit and forms a seal to control the flow of water. The piston is acted upon by an external actuation coupling attached to a spindle of the piston that moves inside a slanted window in the wall of the conduit. In an alternative embodiment, the external actuation coupling is threaded at its inside and engages a helicoid thread attached to the piston which also protrudes through a window in the conduit. In both cases the helicoidal turning movement of the external coupling is transformed into a rising or lowering displacement of the piston to close or open the mouth of the sealing chamber on which the frontal side of the piston head sits.

EP 2 336 610 B1 shows a disposable sterile fluid transfer device in the form of a valve, in particular for use in the pharmaceutical and biopharmaceutical industries. The device includes a body having a bore formed through its interior. Contained within the bore is a movable plunger. An outer cam actuator collar is rotatable relative to the body and causes a ring collar to move axially via a radially outwardly extending cam actuator collar engaging member. The axial movement of the ring collar is transferred to the plunger via a radially inwardly extending plunger engaging member. The engaging members extend in both directions through an aperture in the ring collar. The cam actuator collar is made up of a pair of split collars, and the ring collar is made up of a pair of split rings. The design of the device requires at least three O-rings.

EP 2 060 835 B1 shows a similar fluid transfer device comprising a body and a hollow plunger that is linearly moveable within a bore formed in the body. The body is formed from two sections, a rotating first section and a stationary second section. The bore is also formed of two sections, a rotating bore section which corresponds to the inner wall of the rotating first section, and a stationary bore section which corresponds to the inner wall of the stationary second section. A gland seal is arranged along a length of the plunger. When the first section of the body is rotated, engaging the second section of the body and the plunger, the plunger is driven linearly within the bore, thereby actuating (i.e., opening and closing) the device. Rotation of the plunger is inhibited by a pair of wings extending from the outer wall of the plunger which are received in corresponding pairs of slots formed in the inner wall of the stationary second section of the body. The wings and slots are located within the fluid stream. The design of the device requires that the rotating first section of the body is made from two halves that need to be attached together during assembly.

It is an object of the invention to overcome drawbacks of known valve devices and to provide a compact and reliable valve device with a simple design that can be easily handled by a user and manufactured at low cost.

The above problem is solved by a valve device according to claim 1. Advantageous and expedient embodiments of the invention are apparent from the dependent claims.

The invention provides a valve device, especially for use with a small volume filtration device or for use in a fluid line in a pharmaceutical or biotechnology application. The valve device comprises a housing component including a fluid chamber and a fluid transfer opening for establishing fluid communication with a first external fluid component. The valve device further comprises a valve body received in the housing component. The valve body includes a hollow section, a first end cooperating with the fluid transfer opening inside the housing component, a second end for connecting the valve device to a second external fluid component, at least one first valve body opening connecting the hollow section with the fluid chamber of the housing component, a second valve body opening for fluid transfer from or to the second external fluid component, and a first thread structure formed at an outer wall of the valve body. The valve body is translationally movable along an axis in the housing component between an open position and a closed position. In the closed position of the valve body the second end closes the fluid transfer opening of the housing component. In the open position of the valve body the second end is removed from the fluid transfer opening of the housing component so that a fluid connection is established between the fluid transfer opening and the fluid chamber. The valve device further comprises an actuator ring element for actuating the valve device. The actuator ring element is rotatably mounted on the housing component and includes an accessible structure that can be engaged manually or by a tool to rotate the actuator ring element, and a second thread structure formed at an inner wall of the actuator ring element. The second thread structure engages the first thread structure of the valve body, so that a rotation of the actuator ring element relative to the housing component and the valve body causes the valve body to translationally move in the housing component. The valve device further comprises means for sealing the fluid chamber from the outside of the valve device.

The invention is, inter alia, based on the finding that the mutually engaging thread structures of the actuator ring element and the valve body provide several advantages. First, the thread structures effectively transform a rotational movement of the actuator ring element into a translational (linear) movement of the valve body, which is used to open or close the fluid transfer opening in the housing component of the valve device. Second, compared to known pin/slot constructions or the like, the thread structures allow for a more precise translational movement.

The valve device according to the invention requires only three essential components: the housing component, the valve body and the actuator ring element. These components can be produced at low cost and assembled easily. The valve device is very compact, and one or more valve devices can be installed at inlets and/or outlets of filtration devices, especially small volume filtration devices, in various positions and orientations. It is also possible to install the valve device in pipes or other fluid lines in order to replace clamps or the like for controlling the flow of a fluid. Handling of the valve device is simple and can be accomplished manually or via an automated drive mechanism.

When the valve body of the valve device is in the open position, a fluid connection is established between the fluid transfer opening and the fluid chamber in the housing component. To ensure that the fluid cannot reach critical areas, such as the thread structures or any guiding structures, or even escape the valve device, appropriate sealing is required. According to a preferred embodiment, the means for sealing include at least one O-ring or gasket that is arranged on the valve body and engages an inner wall of the fluid chamber. Of course, the O-ring or gasket can alternatively be arranged on an inner wall of the fluid chamber and engage the valve body. In both cases, the O-ring or gasket forms a movable boundary of the fluid chamber. The means for sealing also prevents any particles from the outside which may have found a way into the second end section of the housing component from entering the fluid chamber and contaminating the fluid.

To further ensure that no fluid gets lost, the hollow section of the valve body, apart from the necessary first valve body opening(s) and the second valve body opening, should otherwise be completely closed. The closed structure of the valve body interior also ensures that no particles from the outside can reach the hollow section of the valve body via any engagement structures or the like.

In order to prevent dust or other unwanted particles from entering the inside of the valve device, including the regions beneath the actuator ring element, sealing can be provided between the valve body and the actuator ring element and between the housing component and the actuator ring element at appropriate locations. Such sealing can be realized with O-rings or gaskets, for example.

To ensure proper function and to avoid faulty operation of the valve device, the open and closed positions of the valve body should be clearly defined, and care should be taken that the valve body is not moved beyond these positions. This can be facilitated by means for limiting the rotational movement of the actuator ring element. Limiting the rotational movement of the actuator ring element means limiting the translational movement of the valve body, due to the concept of transforming rotational movement into translational movement. Therefore, the limits of the rotational movement of the actuator ring element can be set such that the end positions of the actuator ring element correspond to the open and closed positions of the valve body.

According to a preferred embodiment, the means for limiting the rotational movement of the actuator ring element include a dedicated shape, such as a pin or the like, formed on the actuator ring element which interacts with a stop formed on the housing component in an end position of the actuator ring element. Of course, it is also possible to provide the shape on the housing component and the stop on the actuator ring element.

Although the rotational movement of the actuator ring element is transformed into a translational movement of the valve body by the thread structures, it is still possible that the valve body is entrained to some extent and twists relative to the housing component. Therefore, it is advantageous to provide means for preventing twisting of the valve body in the housing component, especially during translational movement of the valve body. When undesired twisting can be entirely avoided, non-twistable connections of the valve device with hard pipes or tubes are possible.

According to a preferred embodiment, the means for preventing twisting of the valve body include at least one rigid protrusion radially extending from the valve body. The protrusion is received in a receptacle formed in the housing component. The receptacle has a circumferential length matching the circumferential length of the protrusion. This ensures that the protrusion (and thus the whole valve body) cannot rotate relative to the housing component. Preferably, the protrusion on the valve body is formed as a pair of wings, and the receptacle in the housing component is formed as a pair of matching slots.

In addition, or as an alternative, to the means for limiting the rotational movement of the actuator ring element, the valve device can comprise means for limiting the translational movement of the valve body. In this case the limits of the translational movement should be set such that they correspond to the open and closed positions of the valve body.

According to a particularly preferred embodiment, the rigid protrusion used for preventing twisting of the valve body relative to the housing component is also used to cooperate with a shoulder formed in the housing component and/or with a shoulder formed in the actuator ring element. The shoulder(s) form(s) a stop or stops for the protrusion to limit the translational movement of the valve body. This means that the protrusion, preferably a pair of wings, fulfills a double function.

It may be desirable that the valve body is installed in the housing component in a predetermined rotational orientation with respect to the axis of the valve device. This can be ensured by matching structures of the valve body and the housing component, allowing installation of the valve body in the housing component only in a predetermined orientation of the valve body.

Such matching structures can be formed by the at least one rigid protrusion of the valve body and the receptacle of the housing component. Accordingly, in this case the protrusion of the valve body and the receptacle of the housing component fulfill yet another function.

According to a particularly advantageous embodiment, the valve device comprises means for preventing translational movement of the actuator ring element relative to the housing component. This means that the actuator ring element does not move together with the valve body in axial directions, but rather always remains at the same axial height, irrespective of the valve position (open, closed, intermediate). This makes automation of the actuation of the valve device easier. Apart from that, operation of the valve device in confined spaces is also made easier.

The valve device according to the invention may comprise means for securing the actuator ring element on the housing component, thus preventing unintended removal of the actuator ring from the housing component.

Preferably, the means for securing the actuator ring element on the housing component include an elastic snap-fit structure formed on at least one non-circumferential portion of the actuator ring element, such as a hook structure engaging an opposite recess in the housing component. "Non-circumferential" means that such portion(s) of the actuator ring element can be easily radially deflected during assembly without a risk of damaging or permanently deforming the actuator ring element. However, it is also possible to have a fully circumferential snap-fit structure.

According to an advantageous embodiment, the means for preventing translational movement of the actuator ring element and the means for securing the actuator ring element on the housing component are the same, thus simplifying the design of the actuator ring element.

Preferably, the actuator ring element, apart from a first ring opening through which the valve body extends and an opposite opening through which the housing component extends, is otherwise completely closed. Accordingly, no particles can penetrate into the valve device through any additional apertures in the actuator ring element.

As mentioned above, the actuator ring element should not have any apertures that do not serve an essential function. Moreover, the outer surfaces of the valve device, including the outer surfaces of the actuator ring element, should be rather smooth and easily wipeable. Moreover, the design of the actuator ring element should be as simple as possible in view of manufacturing (typically injection moulding or 3D-printing). Therefore, in order to meet all these requirements, it may be beneficial to have an actuator ring element composed of two (or even more) separately produced pieces, compared to one complex single-piece actuator ring element. Each individual piece can have a relatively simple design, so that no complex moulding/demoulding tools or 3D-printing techniques are required.

In particular, the actuator ring element may be composed of an inner engagement part including the second thread structure, and a separate cap surrounding the inner engagement part. Especially the cap can be made without any unnecessary apertures and with a wipeable outer surface. Another aspect to be considered is that the inner engagement part and the cap can be made from different materials, according to the different specific functions to be fulfilled by the inner engagement part and the cap, respectively.

According to a preferred design of the actuator ring element, an outer wall of the inner engagement part has several radially protruding ribs extending axially from a radial flange of the inner engagement part, and the cap has an inner structure that matches the ribs of the inner engagement part. This ensures that rotation of the cap, which is engaged by the user or an automated drive mechanism, is transferred to the inner engagement part, which cooperates with the valve body via the thread structures.

Correct assembly of the cap and the inner engagement part can be ensured by matching structures of both pieces, allowing installation of the cap on the inner engagement part only in a predetermined orientation of the cap.

Preferably, the accessible structure of the actuator ring element includes a specially designed gripping structure configured to be engaged manually or by a tool having an engagement structure matching the gripping structure. The latter is important in view of automation of the valve device, i.e. when the tool is part of an automated drive mechanism.

However, the actuator ring element can be made as a single piece, of course, including at least one uninterrupted circumferential portion.

According to a modular concept of the invention, the valve device can comprise a plurality of actuator ring elements with different outer designs. Each of these actuator ring elements can be mounted on the housing component. So the user can choose from various designs of the actuator ring element, depending on the specific use case (manual operation, operation by an automated drive mechanism having a specific engagement structure, etc.).

Further features and advantages of the invention will become apparent from the following description and from the accompanying drawings to which reference is made. In the drawings:
- Figure 1 shows a perspective view of the parts of a first embodiment of the valve device according to the invention;
- Figures 2a and 2b show sectional views of the assembled valve device of Figure 1 in a closed state and in an open state, respectively;
- Figure 3 shows a perspective view of the parts of a second embodiment of the valve device according to the invention; and
- Figures 4a and 4b show sectional views of the assembled valve device of Figure 3 in a closed state and in an open state, respectively.

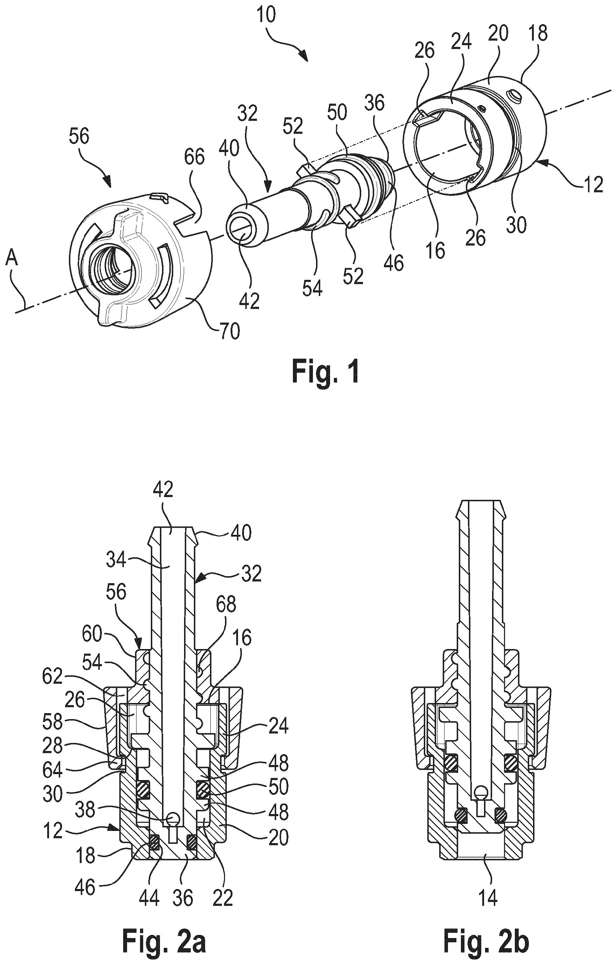

A first embodiment of a valve device 10 designed especially for use with a small volume filtration device or for use in a fluid line in a pharmaceutical or biotechnology application is illustrated in Figures 1 and 2a, 2b. The valve device 10 includes three main parts that will be explained in detail below.

The first main part is a housing component 12 having a basically cylindrical body defining an axis A. The housing component 12 has a fluid transfer opening 14 at a first axial end for establishing fluid communication with a first external fluid component (not shown) and a receiving opening 16 at an opposite second axial end. The first axial end of the housing component 12 can be configured as a connector, or it can be configured for receiving a connector, in order to facilitate the connection with the first external fluid component.

The fluid transfer opening 14 is formed in a first end section 18 of the housing component 12 which has a defined small inner diameter. Adjacent to the first end section 18 is a middle section 20 surrounding a fluid chamber 22 having a defined inner diameter which is larger than that of the first end section 18. The receiving opening 16 is formed in a second end section 24 which basically has the same inner diameter as the fluid chamber 22. Two opposite slots 26 are formed in the inner wall of the second end section 24. The slots 26 are open in the direction towards the receiving opening 16. Their opposite ends are closed and form shoulders 28 at the transition between the second end section 24 and the fluid chamber 22 in the middle section 20 of the housing component 12.

The outer wall of the housing component 12 is generally smooth. A circumferential groove 30 is formed between the middle section 20 and the second end section 24.

The second main part of the valve device 10 is a basically cylindrical valve body 32 having a hollow section 34 extending almost along the entire axial length of the valve body 32. The outer diameter of the valve body 32 is generally constant over its axial length and corresponds to the inner diameter of the fluid transfer opening 14 of the housing component 12.

A first axial end 36 of the valve body 32 is closed, but one or more first valve body openings 38 near the first axial end 36 extend through the sidewall of the valve body 32 into its hollow section 34. The opposite second axial end 40 of the valve body 32 has a second valve body opening 42 and is configured for connecting the valve device 10 to a second external fluid component (not shown). The second axial end 40 of the valve body 32 can be configured as a connector, or it can be configured for receiving a connector, in order to facilitate the connection with the second external fluid component. Apart from the first valve body opening(s) 38 and the second valve body opening 42, the valve body 32 is otherwise completely closed.

Close to the first axial end 36 a circumferential groove 44 is formed in the outer wall of the valve body 32, accommodating a first seal, in particular a first O-ring 46 or a gasket. Further remote from the first axial end 36 a space between two radially protruding circumferential ribs 48 accommodates a second seal, in particular a second O-Ring 50 or a gasket.

Still further remote from the first axial end 36 two opposite wings 52 extend radially from the outer wall of the valve body 32. The wings 52 protrude radially further out from the outer wall than the two circumferential ribs 48. The span of the wings 52 is adapted to the radial depth of the slots 26 formed in the housing component 12. Moreover, the length of the slots 26 in the circumferential direction is also adapted to match the corresponding length of the wings 52.

Still further remote from the first axial end 36 a first thread structure 54 is formed on the outer wall.

The third main part of the valve device 10 is an actuator ring element 56 for actuating the valve device 10. The actuator ring element 56 has a wide first section 58 with an inner diameter adapted to the outer diameter of the second end section 24 of the housing component 12, and a narrow second section 60 with an inner diameter adapted to the outer diameter of the second axial end 40 of the valve body 32. Due to the different diameters of the first and second sections 58, 60, a shoulder 62 is formed between them.

At the free axial end of the wide first section 58 a hook structure 64 extends inwardly from the inner wall of the actuator ring element 56. The inner wall of this axial end with the hook structure 64 is not fully circumferential but interrupted by one or more cutouts 66, resulting in a segmented structure. However, a fully circumferential axial end of the actuator ring element 56 is also possible.

The narrow second section 60 of the actuator ring element 56 includes a second thread structure 68 formed at the inner wall and matching the first thread structure 54 of the valve body 32.

At the outside of the narrow second section 60 a gripping structure 70 is formed at an axial end of the actuator ring element 56. The gripping structure 70 is an accessible structure that can be engaged manually or by a tool of an automated drive mechanism (not shown) having an engagement structure matching the gripping structure 70 of the actuator ring element 56. The gripping structure 70 may include a special profile (not shown here).

The openings at the axial end of the actuator ring element 56 next to the gripping structure 70 are due to the manufacturing process, i.e. they are advantageous for demoulding after injection moulding of the actuator ring element 56.

The valve device 10 is assembled by inserting the first axial end 36 of the valve body 32 into the receiving opening 16 of the housing component 12 in a predefined orientation that allows the wings 52 of the valve body 32 to enter the matching slots 26 formed in the housing component 12. The actuator ring element 56 is then slipped over the second axial end 40 of the valve body 32 and the second end section 24 of the housing component 12 so that the first and second thread structures 54, 68 engage each other and until the hook structure 64 of the actuator ring element 56 engages into the groove 30 of the housing component 12, thus securing the actuator ring element 56 on the housing component 12. The necessary outward deflection of the hook structure 64 before it snaps into the groove 30 is facilitated by the cutouts 66 which prevent the axial end of the wide first section 58 of the actuator ring element 56 from being permanently deformed. After assembly, the actuator ring element 56 is rotatable relative to the housing component 12 and the valve body 32.

By turning the actuator ring element 56 the valve body 32 can be moved along the axis A in the housing component 12 between a closed position shown in Figure 2a and an open position shown in Figure 2b. The translational movement of the valve body 32 is effected by the mutually engaged first and second thread structures 54, 68 of the valve body 32 and of the actuator ring element 56, respectively, which transform the rotational movement of the actuator ring element 56 into a translational movement of the valve body 32.

Since the wings 52 of the valve body 32 exactly fit into the slots 26 of the housing component 12, the valve body 32 is prevented from twisting in the housing component 12. Axial movement of the actuator ring element 56 relative to the housing component 12 is prevented by the snap-fit connection established by the hook structure 64 of the actuator ring element 56 and the groove 30 of the housing component 12. This means that the actuator ring element 56, irrespective of its rotational position, always remains in the same axial position relative to the housing component 12.

When the valve body 32 is moved towards the closed position by turning the actuator ring element 56 in a first direction, the translational movement of the valve body 32 is stopped when its wings 52 hit the shoulders 28 at the bottom of the slots 26. The translational movement of the valve body 32 towards the open position, caused by turning the actuator ring element 56 in an opposite second direction, is stopped when the wings 52 hit the shoulder 62 of the actuator ring element 56 covering the open ends of the slots 26.

In the closed position of the valve body 32 (Figure 2a) the second axial end 40 closes the fluid transfer opening 14 of the housing component 12. More precisely, the first O-ring 46 seals the fluid transfer opening 14 so that there is no fluid communication between the fluid chamber 22 of the housing component 12 and the first external fluid component (not shown) connected at the first end section 18 of the housing component 12. In effect, fluid communication between the first external fluid component and the second external fluid component (not shown, either) connected to the second axial end 40 of the valve body 32 is prevented.

In the open position of the valve body 32 (Figure 2b) the second axial end 40 is removed from the fluid transfer opening 14 of the housing component 12 so that a fluid connection is established between the fluid transfer opening 14 and the fluid chamber 22 of the housing component 12. Due to the first valve body opening(s) 38 fluid communication between the fluid chamber 22 and the hollow section 34 of the valve body 32 is possible. Depending on the flow direction, fluid can either pass from the first external fluid component (not shown) via the fluid chamber 22 and the hollow section 34 through the second valve body opening 42 to the second external fluid component (not shown, either), or vice versa.

It is to be noted that, irrespective of the actual position of the valve body 32, the second O-ring 50 seals the fluid chamber 22 so that no fluid can reach the areas of the wings 52 and the first and second thread structures 54, 68. On the other hand, no contaminants from the outside can pass the second O-ring 50 and reach the fluid chamber 22 in the housing component 12.

It is also to be noted that in the assembled state of the valve device 10, all outer surfaces that are exposed to the environment are rather smooth and can be easily cleaned by simply wiping them, especially when the free axial end of the wide first section 58 of the actuator ring element 56 has no cutouts 66.

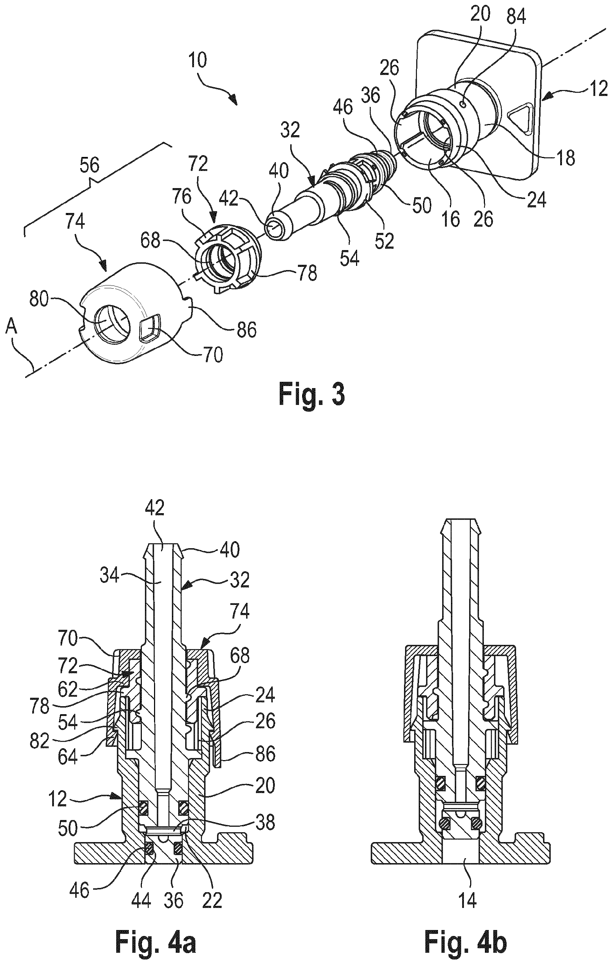

A second embodiment of the valve device 10 is illustrated in Figures 3 and 4a, 4b. The same reference signs are used for the components corresponding to those of the first embodiment, and reference is made in this respect to the explanations above. In the following, only the differences from the first embodiment are explained.

In the second embodiment the actuator ring element 56 is not made as a single part but comprises two separate parts, i.e. an inner engagement part 72 and a cap 74.

The inner engagement part 72 includes the second thread structure 68 formed in its inner wall.

The outer wall of the inner engagement part 72 has several radially protruding ribs 76 extending axially from a radial flange 78 of the inner engagement part 72.

The cap 74 has an inner structure 80 that matches the ribs 76 of the inner engagement part 72.

Formed in the outer sidewall of the cap 74 is a gripping structure 70 that is realized here by two or more recesses. The recesses do not extend completely through the sidewall and can be engaged manually or by a tool of an automated drive mechanism (not shown) having an engagement structure matching the gripping structure 70 of the cap 74.

The cap 74 also has the hook structure 64 formed at one axial end thereof. Unlike in the first embodiment, the section of the cap 74 with the hook structure 64 is not interrupted by any cutouts. However, the hook structure 64 itself is interrupted and thus not completely circumferential.

Instead of a groove 30, the housing component 12 has a reverse hook structure 82 matching the hook structure 64 of the cap 74.

The housing component 12 also has a stop 84 protruding from the outer wall.

The axial end of the cap 74 with the hook structure 64 has a pin 86 that extends in axial direction.

Assembly of the second embodiment is different from the first embodiment in that the inner engagement part 72 is slipped over the second axial end 40 of the valve body 32 first so that the first and second thread structures 54, 68 engage each other and until the flange 78 rests on the second end section 24 of the housing component 12. The cap 74 is then slipped over the inner engagement part 72 in an orientation that allows the inner structure 80 of the cap 74 to engage with the ribs 76 of the inner engagement part 72, until the shoulder 62 of the cap 74 rests on the flange 78 of the inner engagement part 72 and the hook structure 64 of the cap 74 snaps behind the reverse hook structure 82 of the housing component 12. In the assembled state, the cap 74 completely surrounds the inner engagement part 72.

According to an alternative assembly method, the actuator ring element 56 is preassembled by slipping the cap 74 over the inner engagement part 72 before the preassembled actuator ring element 56 is then slipped over the second axial end 40 of the valve body 32 onto the housing component 12 as a whole.

Irrespective of the assembly method, the inner engagement part 72 and the cap 74 can include matching structures that only allow insertion of the inner engagement part 72 into the cap 74 in a predefined orientation of the inner engagement part 72 relative to the cap 74, similar to the wings 52 and slots 26 allowing insertion of the valve body 32 into the housing component 12 in a predefined orientation of the valve body 32 relative to the housing component 12.

Operation of the valve device 10 is the same as in the first embodiment. Due to the ribs 76 of the inner engagement part 72 and the matching inner structure 80 of the cap 74, rotational movement of the cap 74 is transferred to the inner engagement part 72, i.e. inner engagement part 72 is entrained by the cap 74 and the actuator ring element 56 is rotated as a whole. The rotational movement of the actuator ring element 56 is transformed into a translational movement of the valve body 32 along the axis A via the first and second thread structures 54, 68 in the same way as in the first embodiment, while the whole actuator ring element 56, due the thread structures 54, 68 and the snap-fit connection between the actuator ring element 56 and the housing component 12, remains in its axial position relative to the housing component 12, i.e. the actuator ring element 56 is not translationally entrained by the moving valve body 32.

In addition, or as an alternative, to the means for limiting the axial movement of the valve body 32 in the first embodiment (wings 52 and shoulders 28, 62), in the second embodiment the pin 86 of the cap 74 cooperates with the stop 84 of the housing component 12. The cap 74 can be turned in one direction until the pin 86 hits the stop 84 from one side (first end position), and, likewise, the cap 74 can be turned in the opposite direction until the pin 86 hits the stop 84 from the opposite side (second end position), thus defining a maximum angular range of rotation. The first and second thread structures 54, 68 are adapted to this angular range of the actuator ring element 56 so that the valve body 32 assumes the open position and the closed position when the actuator ring element 56 is in the first end position and in the second end position, respectively.

In the assembled state of the valve device 10 all outer surfaces that are exposed to the environment are closed and rather smooth and can be easily cleaned by simply wiping them.

In general, an additional sealing (not shown) can be provided to protect the interior of the valve device 10 from dust or other unwanted particles. In the second embodiment such an additional sealing can be realized with two additional seals such as O-rings. One O-ring can be provided radially between the valve body 12 and the actuator ring element 56 and axially between the upper end of the inner engagement part 72 and the upper end of the cap 74 (with respect to the upright position of the valve device 10 in Figures 4a and 4b), and another O-ring can be provided radially between the housing component 12 and the cap 74 and axially between the reverse hook structure 82 and the receiving opening 26 of the housing component 12.

While two distinct embodiments of the valve device 10 have been described, further embodiments may include combinations of certain features from both embodiments.

The valve device 10 may comprise a variety of different actuator ring elements 56. In particular, the outer design, especially the gripping structure 70, of the actuator ring elements 56 can be adapted to different tools. The user can then select the actuator ring element 56 (or the cap 74) to be mounted depending on whether, and if so, which tool of an automated drive mechanism will be used to turn the actuator ring element 56.

As already mentioned, the valve device 10 is designed especially for use with a small volume filtration device or for use in a fluid line in a pharmaceutical or biotechnology application. However, it is possible to use the valve device 10 in other applications, of course.

### List of Reference Signs

- 10: valve device
- 12: housing component
- 14: fluid transfer opening
- 16: receiving opening
- 18: first end section
- 20: middle section
- 22: fluid chamber
- 24: second end section
- 26: slots
- 28: shoulder
- 30: groove
- 32: valve body
- 34: hollow section
- 36: first axial end
- 38: first valve body opening(s)
- 40: second axial end
- 42: second valve body opening
- 44: groove
- 46: first O-ring
- 48: ribs
- 50: second O-ring
- 52: wings
- 54: first thread structure
- 56: actuator ring element
- 58: first section
- 60: second section
- 62: shoulder
- 64: hook structure
- 66: cutouts
- 68: second thread structure
- 70: gripping structure
- 72: inner engagement part
- 74: cap
- 76: ribs
- 78: flange
- 80: inner structure
- 82: reverse hook structure
- 84: stop
- 86: pin
- A: axis

## Claims

1. A valve device (10), especially for use with a small volume filtration device or for use in a fluid line in a pharmaceutical or biotechnology application,
the valve device (10) comprising a housing component (12) including
a fluid chamber (22), and
a fluid transfer opening (14) for establishing fluid communication with a first external fluid component,
the valve device (10) further comprising a valve body (32) received in the housing component (12), the valve body (32) including
a hollow section (34),
a first end cooperating with the fluid transfer opening (14) inside the housing component (12),
a second end for connecting the valve device (10) to a second external fluid component,
at least one first valve body opening (38) connecting the hollow section (34) with the fluid chamber (22) of the housing component (12),
a second valve body opening (42) for fluid transfer from or to the second external fluid component, and
a first thread structure formed at an outer wall of the valve body (32),
the valve body (32) being translationally movable along an axis in the housing component (12) between an open position and a closed position, wherein
in the closed position of the valve body (32) the second end closes the fluid transfer opening (14) of the housing component (12), and
in the open position of the valve body (32) the second end is removed from the fluid transfer opening (14) of the housing component (12) so that a fluid connection is established between the fluid transfer opening (14) and the fluid chamber (22),
the valve device (10) further comprising an actuator ring element (56) for actuating the valve device (10), the actuator ring element (56) being rotatably mounted on the housing component (12) and including
an accessible structure that can be engaged manually or by a tool to rotate the actuator ring element (56), and
a second thread structure formed at an inner wall of the actuator ring element (56), the second thread structure engaging the first thread structure of the valve body (32),
so that a rotation of the actuator ring element (56) relative to the housing component (12) and the valve body (32) causes the valve body (32) to translationally move in the housing component (12),
the valve device (10) further comprising means for sealing the fluid chamber (22) from the outside of the valve device (10).

2. The valve device (10) according to claim 1, **characterized in that** the means for sealing include at least one O-ring (50) or gasket, arranged on the valve body (32) and engaging an inner wall of the fluid chamber (22) of the housing component (12), or arranged on an inner wall of the fluid chamber (22) and engaging the valve body (32), thus forming a movable boundary of the fluid chamber (22).

3. The valve device (10) according to claim 1 or 2, **characterized in that** the valve device (10) comprises means for limiting the rotational movement of the actuator ring element (56).

4. The valve device (10) according to any of the preceding claims, **characterized in that** the valve device (10) comprises means for preventing twisting of the valve body (32) in the housing component (12), especially during translational movement of the valve body (32).

5. The valve device (10) according to claim 4, **characterized in that** the means for preventing twisting of the valve body (32) include at least one rigid protrusion, preferably a pair of wings (52), radially extending from the valve body (32) which is received in a receptacle, preferably a pair of slots (26), formed in the housing component (12), the receptacle having a circumferential length matching the circumferential length of the protrusion.

6. The valve device (10) according to any of the preceding claims, **characterized in that** the valve device (10) comprises means for limiting the translational movement of the valve body (32).

7. The valve device (10) according to claims 5 and 6, **characterized in that** the at least one protrusion of the valve body (32) also cooperates with a shoulder (28) formed in the housing component (12) and/or with a shoulder (62) formed in the actuator ring element (56), the shoulder(s) (28, 62) forming a stop or stops for the protrusion to limit the translational movement of the valve body (32).

8. The valve device (10) according to any of the preceding claims, **characterized in that** the valve body (32) and the housing component (12) include matching structures allowing installation of the valve body (32) in the housing component (12) only in a predetermined orientation of the valve body (32).

9. The valve device (10) according to any of claims 5 to 7 and claim 8, **characterized in that** the matching structures are formed by the at least one protrusion of the valve body (32) and the receptacle of the housing component (12).

10. The valve device (10) according to any of the preceding claims, **characterized in that** the valve device (10) comprises means for preventing translational movement of the actuator ring element (56) relative to the housing component (12).

11. The valve device (10) according to any of the preceding claims, **characterized in that** the valve device (10) comprises means for securing the actuator ring element (56) on the housing component (12).

12. The valve device (10) according to claim 10 and claim 11, **characterized in that** the means for preventing translational movement of the actuator ring element (56) and the means for securing the actuator ring element (56) are the same.

13. The valve device (10) according to any of the preceding claims, **characterized in that** the actuator ring element (56), apart from a first ring opening through which the valve body (32) extends and an opposite opening through which the housing component (12) extends, is otherwise completely closed.

14. The valve device (10) according to any of the preceding claims, **characterized in that** the actuator ring element (56) comprises an inner engagement part (72) including the second thread structure (68), and a separate cap (74) surrounding the inner engagement part (72).

15. The valve device (10) according to any of the preceding claims, **characterized in that** the accessible structure of the actuator ring element (56) includes a gripping structure (70) configured to be engaged manually or by a tool having an engagement structure matching the gripping structure (70), the tool preferably being part of an automated drive mechanism.
